# EUROPEAN PATENT SPECIFICATION

(11) **EP 0 554 319 B1**
(45) Date of publication and mention of the grant of the patent: **27.12.2000**
(21) Application number: 91918844.1
(22) Date of filing: 28.10.1991
(51) Int. Cl.: A61K 35/78, A61K 35/74

(54) **ANTIMICROBIAL COMPOSITION**
ANTIMIKROBIELLE ZUSAMMENSETZUNG
COMPOSITION ANTIMICROBIENNE

(30) Priority: 26.10.1990 GB 9023337
(43) Date of publication of application: 11.08.1993
(73) Proprietor: INTERPRISE LIMITED, Baglan, Port Talbot, West Glamorgan SA12 7DJ (GB)
(72) Inventor: PLUMMER, Nigel, Swansea, West Glamorgan SA6 7DZ (GB)
(74) Representative: Austin, Hedley William
(86) International application number: GB9101885
(87) International publication number: WO9207575

(56) References cited:
- EP-A- 0 302 300
- Dialog Information Services, File 350, WPI 74-80, Dialog accession no. 000988351, LEHNER AG: "Stabilized garlic powder - produced by powdering deep-frozen garlic and freeze-drying", CH 540013, A, 7344 (Basic)
- Dialog Information Services, File 350, WPI 74-80, Dialog accession no. 000889166, FORM E: "Stable garlic powder - produced by deep freezing", DE 2101880, A, 7231 (Basic)
- Dialog Information Services, File 155, Medline 66- 91, Dialog accession no. 03049637, Tynecka Z et al: "The fungistatic activity of garlic (Allium sativum L.) in vitro", Ann Univ Mariae Curie SklodowskaMed 1975, 30 p 5-13
- Dialog Information Services, File 351, WPI 81-91, Dialog accession no. 004840940, RIKEN KAGAKU KOGYO: "Growth accelerator for bifidobacterium etc. compri-ses garlic extracts e.g. oxoamidine(rtm), peptone sodium chloride which are added to culture", JP 61260023, A, 861118, 8652 (Basic)
- Dialog Information Services, File 155, Medline 66- 91, Dialog accession no. 03049637, Tynecka Z et al: "The fungistatic activity of garlic (Allium sativum L.) in vitro", Ann Univ Mariae Curie Sklodowska Med 1975, 30 p 5-13

## Description

The present invention is concerned with antimicrobial compositions, and the use thereof in the health/nutrition industry as dietary supplements, and as silage treatment agents.

It is known that antimicrobial materials can be extracted from the plant family Allium (which includes edible plants such as onions, chives, shallots, leek and garlic). The antimicrobial properties of garlic are well documented and garlic juice has been shown to inhibit the growth of a variety of pathogenic micro-organisms including Staphylococcus, Klebsiella, Proteus, Escherichia coli and Salmonella.

The antibacterial activity of garlic is attributed very largely to a compound known as allicin. About 0.24% (w/w) of each garlic clove consists of a compound known as alliin which is a non-odoriferous derivative of the amino acid cysteine. Intracellularly separated from the alliin in the garlic clove is an enzyme known as alliinase.

When the garlic clove is crushed, the alliinase comes into contact with the alliin to produce the odorous, unstable, water-soluble substance known as allicin (diallyl thiosulphinate).

Allicin decomposes readily to form a variety of intermediate products ultimately consisting of a mixture of allyl sulphides. This degradation process occurs at room temperature but is very much faster at elevated temperatures. The degradation products of allicin (i.e. the diallyl sulphides) have very little antimicrobial activity.

The mode of action of the allicin is largely unknown, but it is thought to act by blocking important metabolic enzymes, particularly those containing reactive SH groups. Alternatively, it may act by disrupting the microbial cell metabolism by interfering with protein function or by binding to cysteine and glutathione and inhibiting their activity.

The antifungal activity of garlic appears to be a combination of the effects of allicin and a specifically antifungal breakdown product of allicin known as ajoene. It has been found that lipid synthesis in C. albicans can be completely inhibited in the presence of garlic extract.

Dried (or freeze-dried) garlic powder is also known, which is prepared by crushing garlic cloves and then drying the crushed material. This results in reaction of alliin with alliinase to produce allicin.

We have now discovered that improved antimicrobial materials can be obtained from plant sources if special procedures are adopted which have been found to avoid reaction between alliin and alliinase.

The present invention provides, for use of (a) micro-organisms capable of propagation in the gastro-intestinal tract of an animal, the micro-organisms being of any of the genera Lactobacillus, Enterococcus or Pediococcus, and (b) antimicrobial material which:
(i) is derived from the plant family Allium
(ii) comprises alliin and alliinase and is substantially free of allicin, and
(iii) is simultaneously capable of releasing allicin in the tract to selectively attack pathogenic micro-organisms in the tract while selectively not attacking the non-pathogenic micro-organisms in the tract,
in the manufacture of simultaneously or successively orally administrable compositions for promoting colonisation of the gastro-intestinal tract of an animal.

The composition and/or the antimicrobial material preferably contains a carrier which is substantially free of interaction with either the non-pathogenic micro-organisms or with the antimicrobial material. The carrier may be the antimicrobial material, the non-pathogenic micro-organisms, or another material which does not substantially interact with either the antimicrobial material or with the non-pathogenic micro-organisms.

The supplement is preferably used in the manufacture of a material for oral therapeutic treatment of microbial mediated diseases of the gastro-intestinal tract. Examples of such diseases of the gastrointestinal tract, include enteric disease caused by E. coli, rotavirus and Candida spp.

The composition comprising non-pathogenic micro-organisms used according to the present invention provides a source of beneficial micro-organisms which will effectively compete with any potential pathogens present in the gastrointestinal tract, and is therefore capable of colonising the small intestine of the host when the composition is used for administration to animals.

The gastrointestinal tract of the neonatal animal is sterile at birth but it rapidly becomes colonised by the micro-organisms prevailing in the natural environment. It is essential that the organisms that colonise the gastrointestinal tract at that stage are the beneficial organisms which will effectively prevent the establishment of pathogens. However, the pathogens tend to grow more rapidly than the beneficial organisms and this is one reason why disease occurs in young animals.

Preferably the microbial component present in the composition according to the invention comprises at least one strain of a host-specific, non-pathogenic Gram-positive bacteria which:
1. attaches to the epithelial tissue of the small intestine of the host;
2. is resistant to at least 3% (w/w) bile salts;
3. has a doubling time of less than two hours in vitro; and
4. is a homo- or hetero-fermentative lactic acid producer with greater than 40% by molarity of the acid produced thereby being lactic acid.

The non-pathogenic microorganisms preferably substantially retain their stability and characteristic features after being subjected to a typical production strategy of fermentation, centrifugation and spray- or freeze-drying. The micro-organisms are combined according to the invention with the antimicrobial material in such a way that allicin production is not triggered until the supplement is put into use.

Preferred micro-organisms for use according to the invention are of the species Lactobacilius acidophilus or Lactobacillus plantarum.

The antimicrobial activity of the antibacterial material used according to the invention has been found to be highly selective. We have found that there is no adverse effect on selected members of the beneficial lactobacillus microbial population of the gastrointestinal tract.

The antimicrobial material is typically obtained by drying of substantially the entire bulb of the relevant Allium species (which includes many well known edible plants such as onions, chives, shallots, leeks and garlic). The preferred source of the plant extract is Allium sativum (garlic).

It is preferred that fresh substantially whole garlic cloves are freeze dried to produce garlic granules or powder, for use according to the invention. The freeze drying process entails the rapid freezing of the substantially whole cloves followed by gently drying from that frozen state at temperatures between -25 and -5°C.

In the use according to the invention, more than one species of micro-organism may be present. The dosage of micro-organisms depends largely on the target host and the treatment required, but the dosage of micro-organisms in any 24 hours period is typically from 1 x 10⁷ to 1 x 10¹². The dosage of the antimicrobial material also depends upon the target host and treatment required, but the dosage of fresh garlic equivalent administered over a 24 hour period is generally between 1 and 10,000mg. The supplement may be provided in any suitable form, such as a powder, in powder tablet, capsule or similar form, or as a powder which is reconstituted with water.

The supplement may be used as a kit comprising a first receptacle containing an antimicrobial material derived from the plant family Allium, and a second receptacle containing at least one species of non-pathogenic micro-organisms of any of the genera Lactobacillus, Enterococcus or Pediococcus, in which the antimicrobial material comprises alliin and alliinase and is substantially free of allicin, and in which the antimicrobial material being simultaneously capable of selectively inhibiting propagation of pathogenic micro-organisms present in said tract and selectively permitting propagation of said non-pathogenic micro-organisms in said tract.

It is also intended that lower dosage levels (sub-therapeutic amounts) may be used according to the present invention as a growth promoting agent, in a similar way to the way in which sub-therapeutic levels of antibiotics are currently used to perform such a function.

The supplement may be used where physiological, emotional or environmental stresses are placed on the patient which profoundly alters the balance of the microflora. This may include events such as post-antibiotic therapy, post-drug therapy and menstruation in human cases, and weaning in the case of domestic animals.

In addition to its activity against pathogenic bacteria, the supplement used according to the invention has antiviral properties and in vitro activity against influenza B virus. This is particularly important in view of the fact that a large percentage of the cases of diarrhoea in young animals have been attributed to strains of rotavirus rather that to bacterial sources.

The following Examples are given by way of illustration only.

### EXAMPLE 1

Whole garlic cloves were peeled to remove the brittle outer skin. The clove was left entirely intact (cutting the scar causes some allicin to be produced). The cloves were then frozen to -30°C. Primary freeze drying took place with the product at temperatures between -25°C and -5°C.

When the primary freeze drying phase was completed, the temperature of the garlic cloves rose rapidly to +20°C where it remained whilst secondary drying took place to remove desorbed water. This took approximately 2 hours, the entire drying process taking between 12 and 24 hours.

The resulting freeze-dried powder contained 1.55% alliin and had allicin potency (as measured by the size of zone of inhibition of the yeast Candida Albicans) of 15mm.

Similar results, but with slightly less allicin potency, were obtained by cutting off the scar at the end of each clove to accelerate moisture loss during the freeze-drying process.

Again similar results were obtained by cryogenically crushing, dicing or milling of the frozen whole cloves, reaction between alliin and alliinase being substantially prevented by ensuring that the temperatures did not exceed 20°C at any point.

The results achieved using freeze-dried whole garlic cloves are compared with those from crushed garlic in the following table.

| | Alliin (Micrograms/gram Powder) | Allicin Potency |
|---|---|---|
| Whole cloves freeze dried (according to the invention) | 1.55% | 15 |
| Crushed garlic 4°C 30 mins Freeze Dried | 0.42% | 13 |
| Crushed garlic 4°C 2 hours Freeze dried | 0.11% | 12 |
| Crushed garlic 4°C 24 hours Freeze dried | Trace | 9.5 |
| Crushed garlic 20°C 30 mins Freeze dried | 0.04% | 11 |
| Crushed garlic 20°C 2 hours Freeze dried | Trace | 10 |
| Crushed garlic 20°C 24 hours Freeze dried | Trace | 6 |

The above table demonstrates the rapid reduction in the level of alliin after disintegration of a garlic clove, and the concomitant decrease on the allicin potency as demonstrated by a bioassay method against the yeast Candida albicans.

The results show that allicin is rapidly produced from alliin and alliinase even at low temperatures. Also the antimicrobial activity of the preparation is decreased after allicin is formed and this decrease is dependent both upon time and temperature.

In the extreme cases above, garlic cloves which were crushed and kept for 24 hours at 20°C before freeze drying had only 40% of the activity of garlic which was freeze dried as the whole clove.

The Allicin potency (measured in mm) was assayed as follows:

Equidistant wells were bored in agar plates containing MRS (de Mann, Rogosa and Sharpe) medium. The plates were then seeded with a lawn of Candida Albicans.

An overnight mixture of the organism was prepared and the agar plates were flooded with standardised concentrations of the mixture and the excess culture was removed. The lawns of the various cultures were allowed to dry and then the garlic preparations (at 20 mg/ml) were added to the wells in equal concentrations.

The efficacy, or allicin potency, was detected as a zone of clearing around the well containing the garlic and the comparative activities could be assessed by the diameters of the zones (in mm) in inhibition.

The test was repeated for several other micro-organisms, using freeze dried whole garlic cloves according to the invention, at 50 mg/ml; the results are given in the following table:

| Organism | Diameter of Zone (mm) |
|---|---|
| E. coli | 56 |
| C. albicans | 42 |
| S. faecium | No inhibition |
| S. aureus | 40 |
| L. acidophilus | No inhibition |
| L. plantarum | No inhibition |

This showed that the freeze dried garlic had a significant inhibitory effect on the growth of the pathogenic organisms viz. E. coli, C. albicans and Staph aureus. However, the freeze dried garlic preparation did not have any adverse effects on the growth of "beneficial" intestinal organisms such as L. acidophilus,S. faecium or L. plantarum and can therefore be combined therewith to produce compositions according to the invention.

The organisms L. acidophilus and L. plantarum referred to above were isolated from the pig's gut were identified and the Gram positive organisms were selected for further study.

The chosen organisms were put through a screening procedure and five strains of Lactobacillus were finally selected on the basis of their performance. These strains were not fully identified but were:
Lactobacillus acidophilus
Lactobacillus delbruekii
Lactobacillus plantarum
Lactobacillus sp
Lactobacillus sp

On the basis of these results, the organisms were subjected to the production regime and two of the strains showed good survival through the process; these were the L. acidophilus and L. plantarum referred to above.

### EXAMPLE 2

### The Antimicrobial effect of the Composition In Vitro.

The effects of the composition were tested in vitro using the plate/well method previously described. The composition was prepared according to the formulation used for field trial studies and was added to the wells directly. The antimicrobial activity was tested against C. albicans (which was used as the reference organism for testing antimicrobial activity) and E. coli, which is a strain known to be pathogenic to pigs and so provides a good indication of the potential activity of the composition.

| Organism | Diameter of Zone (mm) |
|---|---|
| C. albicans | 15 |
| E. coli | 9 |
| L. acidophilus | No inhibition |
| L. plantarum | No inhibition |

The zones observed during this study were smaller than those observed previously because the garlic concentration (of the composition) was 20 mg/ml compared with the 50 mg/ml used in the previous study.

### EXAMPLE 3

### Effect of strains of Lactobacillus on the growth of C. albicans In Vitro.

It is very difficult to demonstrate the potentially inhibitory activity of Lactobacillus in the laboratory. However, a system has been developed which involved the growth of various strains of Lactobacillus at 30°C for 24 hours and then spinning down to obtain the supernatants which were filter sterilised and used as the aqueous phase for fresh batches of media. The plates which were prepared were inoculated with a pathogenic strain of Candida and the effects of the Lactobacillus growth media on the growth of the yeast were observed.

The results are shown in the following table:

| Organism | Inhibition of Growth (%) |
|---|---|
| L 1 | 40 |
| L 4 | 58 |
| L 5 | 0 |
| L 6 | 69 |
| L 7 | 99 |
| L 8 | 99 |
| L 9 | 100 |
| L10 | 98 |
| L11 | 0 |
| Acidic pH | 63 |

As can be seen, using this modified in vitro method for assessing the inhibition of C. albicans, several strains of Lactobacillus can inhibit the growth of the pathogenic yeast.

## Claims

1. Use of (a) micro-organisms capable of propagation in the gastro-intestinal tract of an animal, said micro-organisms being of any of the genera Lactobacillus, Enterococcus or Pediococcus, and (b) antimicrobial material which:
(i) is derived from the plant family Allium
(ii) comprises alliin and alliinase and is substantially free of allicin, and
(iii) is simultaneously capable of releasing allicin in said tract to selectively attack pathogenic micro-organisms in said tract while selectively not attacking said non-pathogenic micro-organisms in said tract,
in the manufacture of simultaneously or successively orally administrable compositions for promoting colonisation of the gastro-intestinal tract of an animal.

2. Use according to claim 1, wherein said micro-organisms and/or said antimicrobial material are together with a carrier which is substantially free of interaction either with said non-pathogenic micro-organisms or with said anti-microbial material.

3. Use according to claim 1 or 2, said compositions are formulated for oral therapeutic treatment of microbial mediated diseases of said tract.

4. Use according to any of claims 1 to 3, wherein said non-pathogenic micro-organisms have the following characteristics:
(a) they have the ability to attach to the epithelial tissue of the small intestine of said animal;
(b) they have resistance to at least 3% (w/w) bile salts;
(c) they have a doubling time of less than two hours in vitro; and
(d) they are homo - or hetero - fermentative lactic acid producers with greater than 40% by molarity of the acid produced thereby being lactic acid.

5. Use according to any of claims 1 to 3, wherein said Lactobacillus comprise Lactobacillus acidophilus or Lactobacillus plantarum.

6. Use according to any of claims 1 to 3, wherein said Enterococcus comprise Enterococcus faecium.

7. Use according to any of claims 1 to 6, wherein said antimicrobial material is obtained by freeze drying substantially whole cloves of plants of said family.

8. Use according to claim 7, wherein said cloves are of Allium sativum.

## Patentansprüche

1. Verwendung von (a) im Gastrointenstinaltrakt eines Tieres vermehrungsfähigen Mikroorganismen, die zu den Genera Lactobacillus, Enterococcus oder Pediococcus zählen, und (b) eines antimikrobiellen Materials, das
(i) aus Pflanzen der Familie Allium gewonnen ist,
(ii) Alliin und Alliinase umfaßt und im wesentlichen frei ist von Allicin, und das
(iii) gleichzeitig in der Lage ist, Allicin in den Gastrointestinaltrakt abzugeben, um dort selektiv pathogene Mikroorganismen anzugreifen und die nicht-pathogenen Mikroorganismen nicht anzugreifen,
zur Herstellung von gleichzeitig oder nacheinander oral verabreichbaren Zusammensetzungen zur Förderung der Besiedlung des Gastrointestinaltraktes eines Tieres.

2. Verwendung gemäß Anspruch 1, wobei die Mikroorganismen und/oder das antimikrobielle Material zusammen mit einem Träger vorliegen, der im wesentlichen weder mit den nicht-pathogenen Mikroorganismen noch mit dem antimikrobiellen Material wechselwirkt.

3. Verwendung nach Anspruch 1 oder 2, wobei die Zusammensetzungen zur oralen therapeutischen Behandlung von mikrobiell verursachten Krankheiten des Gastrointestinaltraktes formuliert sind.

4. Verwendung nach einem der Ansprüche 1 bis 3, wobei die nicht-pathogenen Mikroorganismen folgende Eigenschaften aufweisen:
(a) Sie sind in der Lage, am Dünndarm-Epithelgewebe des Tieres anzuhaften;
(b) sie sind gegen mindestens 3 Gew.-% Gallensalze (bile salts) resistent;
(c) sie haben eine Verdopplungszelt von weniger als zwei Stunden in vitro; und
(d) sie sind homo- oder heterofermentative Milchsäureproduzenten, wobei mehr als 40 Mol-% der hergestellten Säuren Milchsäure ist.

5. Verwendung nach einem der Ansprüche 1 bis 3, wobei der Lactobacillus Lactobacillus acidophilus oder Lactobacillus plantarum umfaßt.

6. Verwendung nach einem der Ansprüche 1 bis 3, wobei der Enterococcus Enterococcus faecium umfaßt.

7. Verwendung nach einem der Ansprüche 1 bis 6, wobei das antimikrobielle Material unter Gefriertrocknung im wesentlichen ganzer Zehen (cloves) von Pflanzen der genannten Familie erhalten wird.

8. Verwendung nach Anspruch 7, wobei die Zehen solche von Allium sativum sind.

## Revendications

1. Utilisation (a) de micro-organismes susceptibles de se propager dans les voies gastro-intestinales d'un animal, lesdits micro-organismes étant de l'un quelconque des genres Lactobacillus, Enterococcus ou Pediococcus, et (b) de matière antimicrobienne qui : (i) est dérivée de la famille de plantes Allium (ii) comprend de l'alliine et de l'alliinase et est essentiellement dépourvue d'allicine, et (iii) est en même temps susceptible de libérer de l'allicine dans lesdites voies pour attaquer de manière sélective des micro-organismes pathogènes dans lesdites voies tout en n'attaquant pas de manière sélective lesdits micro-organismes non pathogènes dans lesdites voies, dans la fabrication de compositions pouvant être administrées par voie orale de façon simultanée ou consécutive pour favoriser la colonisation des voies gastro-intestinales d'un animal.

2. Utilisation selon la revendication 1, dans laquelle lesdits micro-organismes et/ou ladite matière antimicrobienne sont associés à un excipient qui est essentiellement exempt d'interaction avec lesdits micro-organismes non pathogènes ou avec ladite matière antimicrobienne.

3. Utilisation selon la revendication 1 ou 2, lesdites compositions étant formulées pour le traitement thérapeutique par voie orale des maladies d'origine microbienne desdites voies.

4. Utilisation selon l'une quelconque des revendications 1 à 3, dans laquelle lesdits micro-organismes non pathogènes présentent les caractéristiques suivantes :
(a) ils ont la capacité de se fixer au tissu épithélial de l'intestin grêle dudit animal ;
(b) ils résistent à au moins 3% (poids/poids) de sels bilaires ;
(c) ils ont un temps de doublement inférieur à deux heures in vitro ; et
(d) ce sont des producteurs d'acide lactique homo - ou hétéro - fermentaire, plus de 40% en moles de l'acide ainsi produit étant de l'acide lactique.

5. Utilisation selon l'une quelconque des revendications 1 à 3, dans laquelle ledit Lactobacillus comprend Lactobacillus acidophilus ou Lactobacillus plantarum.

6. Utilisation selon l'une quelconque des revendications 1 à 3, dans laquelle ledit Enterococcus comprend Enterococcus faecium.

7. Utilisation selon l'une quelconque des revendications 1 à 6, dans laquelle ladite matière antimicrobienne est obtenue par lyophilisation essentiellement de gousses entières de plantes de ladite famille.

8. Utilisation selon la revendication 7, dans laquelle lesdites gousses proviennent de Allium sativum.
